(19) Europäisches Patentamt / European Patent Office / Office européen des brevets

(11) EP 2 822 587 B1

(12) **EUROPEAN PATENT SPECIFICATION**

(45) Date of publication and mention of the grant of the patent:
**03.02.2016 Bulletin 2016/05**

(51) Int Cl.:
*A61K 9/08* (2006.01)  *A61K 39/395* (2006.01)
*A61K 9/00* (2006.01)  *A61K 47/10* (2006.01)
*A61K 47/18* (2006.01)  *A61K 47/26* (2006.01)
*C07K 16/18* (2006.01)

(21) Application number: **13707176.7**

(22) Date of filing: **05.03.2013**

(86) International application number:
**PCT/EP2013/054313**

(87) International publication number:
**WO 2013/131866 (12.09.2013 Gazette 2013/37)**

(54) **ABETA ANTIBODY FORMULATION**

ABETA-ANTIKÖRPERFORMULIERUNG

FORMULATION D'ANTICORPS ANTI-PEPTIDE ABÊTA

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.03.2012 EP 12158602**

(43) Date of publication of application:
**14.01.2015 Bulletin 2015/03**

(73) Proprietor: **F.Hoffmann-La Roche AG
4070 Basel (CH)**

(72) Inventors:
• **GOLDBACH, Pierre
68100 Mulhouse (FR)**
• **MAHLER, Hanns-Christian
4054 Basel (CH)**
• **MUELLER, Robert
4058 Basel (CH)**

(74) Representative: **Küng, Peter
F. Hoffmann-La Roche AG
Patent Department
Grenzacherstrasse 124
4070 Basel (CH)**

(56) References cited:
**WO-A1-2007/068429    WO-A1-2008/071394**

• **MANNING M C ET AL: "STABILITY OF PROTEIN PHARMACEUTICALS", PHARMACEUTICAL RESEARCH, KLUWER ACADEMIC PUBLISHERS, NEW YORK, NY, US, vol. 6, no. 11, 1 November 1989 (1989-11-01), pages 903-918, XP000646809, ISSN: 0724-8741, DOI: 10.1023/A:1015929109894 cited in the application**

EP 2 822 587 B1

SEQUENCE LISTING

<110> F. Hoffmann-La Roche AG

<120> Antibody formulation

<130> 27381 WO

<150> EP12158602.8
<151> 2012-03-08

<160> 11

<170> PatentIn version 3.5

<210> 1
<211> 41
<212> PRT
<213> Artificial Sequence

<220>
<223> Abeta 42 peptide

<400> 1

```
Asp Ala Glu Phe Arg His Asp Ser Gly Tyr Glu Val His His Gln Lys
1               5               10              15
```

```
Leu Val Phe Phe Ala Glu Asp Val Gly Ser Asn Lys Gly Ala Ile Ile
            20              25              30
```

```
Gly Leu Met Val Gly Gly Trp Ile Ala
        35              40
```

<210> 2
<211> 126
<212> PRT
<213> Artificial Sequence

<220>
<223> VH domain Abeta Antibody

<400> 2

```
Gln Val Glu Leu Val Glu Ser Gly Gly Gly Leu Val Gln Pro Gly Gly
1               5               10              15
```

```
Ser Leu Arg Leu Ser Cys Ala Ala Ser Gly Phe Thr Phe Ser Ser Tyr
            20              25              30
```

```
Ala Met Ser Trp Val Arg Gln Ala Pro Gly Lys Gly Leu Glu Trp Val
        35              40              45
```

```
Ser Ala Ile Asn Ala Ser Gly Thr Arg Thr Tyr Tyr Ala Asp Ser Val
    50              55              60
```

```
Lys Gly Arg Phe Thr Ile Ser Arg Asp Asn Ser Lys Asn Thr Leu Tyr
65              70              75              80
```

```
Leu Gln Met Asn Ser Leu Arg Ala Glu Asp Thr Ala Val Tyr Tyr Cys
            85              90              95
```

```
Ala Arg Gly Lys Gly Asn Thr His Lys Pro Tyr Gly Tyr Val Arg Tyr
        100             105             110
```

```
Phe Asp Val Trp Gly Gln Gly Thr Leu Val Thr Val Ser Ser
        115             120             125
```

<210> 3
<211> 110
<212> PRT
<213> Artificial Sequence

<220>
<223> VL domain Abeta Antibody

<400> 3

Asp Ile Val Leu Thr Gln Ser Pro Ala Thr Leu Ser Leu Ser Pro Gly
1 5 10 15

Glu Arg Ala Thr Leu Ser Cys Arg Ala Ser Gln Ser Val Ser Ser Ser
20 25 30

Tyr Leu Ala Trp Tyr Gln Gln Lys Pro Gly Gln Ala Pro Arg Leu Leu
35 40 45

Ile Tyr Gly Ala Ser Ser Arg Ala Thr Gly Val Pro Ala Arg Phe Ser
50 55 60

Gly Ser Gly Ser Gly Thr Asp Phe Thr Leu Thr Ile Ser Ser Leu Glu
65 70 75 80

Pro Glu Asp Phe Ala Thr Tyr Tyr Cys Leu Gln Ile Tyr Asn Met Pro
85 90 95

Ile Thr Phe Gly Gln Gly Thr Lys Val Glu Ile Lys Arg Thr
100 105 110

<210> 4
<211> 10
<212> PRT
<213> Artificial Sequence

<220>
<223> VH CDR1 Abeta Antibody

<400> 4

Gly Phe Thr Phe Ser Ser Tyr Ala Met Ser
1 5 10

<210> 5
<211> 17
<212> PRT
<213> Artificial Sequence

<220>
<223> VH CDR2 Abeta Antibody

<400> 5

Ala Ile Asn Ala Ser Gly Thr Arg Thr Tyr Tyr Ala Asp Ser Val Lys
1 5 10 15

Gly

<210> 6

<211> 17
<212> PRT
<213> Artificial Sequence

<220>
<223> VH CDR3 Abeta Antibody

<400> 6

```
Gly Lys Gly Asn Thr His Lys Pro Tyr Gly Tyr Val Arg Tyr Phe Asp
1               5                   10                  15

Val
```

<210> 7
<211> 12
<212> PRT
<213> Artificial Sequence

<220>
<223> VL CDR1 Abeta Antibody

<400> 7

```
Arg Ala Ser Gln Ser Val Ser Ser Ser Tyr Leu Ala
1               5                   10
```

<210> 8
<211> 7
<212> PRT
<213> Artificial Sequence

<220>
<223> VL CDR2 Abeta Antibody

<400> 8

```
Gly Ala Ser Ser Arg Ala Thr
1               5
```

<210> 9
<211> 8
<212> PRT
<213> Artificial Sequence

<220>
<223> VL CDR3 Abeta Antibody

<400> 9

```
Leu Gln Ile Tyr Asn Met Pro Ile
1               5
```

<210> 10
<211> 459
<212> PRT
<213> Artificial Sequence

<220>
<223> Heavy chain Abeta Antibody

<400> 10

```
Gln Val Glu Leu Val Glu Ser Gly Gly Gly Leu Val Gln Pro Gly Gly
1               5               10              15

Ser Leu Arg Leu Ser Cys Ala Ala Ser Gly Phe Thr Phe Ser Ser Tyr
            20              25              30

Ala Met Ser Trp Val Arg Gln Ala Pro Gly Lys Gly Leu Glu Trp Val
            35              40              45

Ser Ala Ile Asn Ala Ser Asn Ala Ser Gly Thr Arg Thr Tyr Tyr Ala
        50              55              60

Asp Ser Val Lys Gly Arg Phe Thr Ile Ser Arg Asp Asn Ser Lys Asn
65              70              75              80

Thr Leu Tyr Leu Gln Met Asn Ser Leu Arg Ala Glu Asp Thr Ala Val
            85              90              95

Tyr Tyr Cys Ala Arg Gly Lys Gly Asn Thr His Lys Pro Tyr Gly Tyr
            100             105             110

Val Arg Tyr Phe Asp Val Trp Gly Gln Gly Thr Leu Val Thr Val Ser
            115             120             125

Ser Ala Ser Thr Lys Gly Pro Ser Val Phe Pro Leu Ala Pro Ser Ser
```

                    130                     135                          140

Lys Ser Thr Ser Gly Gly Thr Ala Ala Leu Gly Cys Leu Val Lys Asp
145                 150                 155                 160

Tyr Phe Pro Glu Pro Val Thr Val Ser Trp Asn Ser Gly Ala Leu Thr
                165                 170                 175

Ser Gly Val His Thr Phe Pro Ala Val Leu Gln Ser Ser Gly Leu Tyr
                180                 185                 190

Ser Leu Ser Ser Val Val Thr Val Pro Ser Ser Ser Leu Gly Thr Gln
                195                 200                 205

Thr Tyr Ile Cys Asn Val Asn His Lys Pro Ser Asn Thr Lys Val Asp
    210                 215                 220

Lys Lys Val Glu Pro Lys Ser Cys Asp Lys Thr His Thr Cys Pro Pro
225                 230                 235                 240

Cys Pro Ala Pro Glu Leu Leu Gly Gly Pro Ser Val Phe Leu Phe Pro
                245                 250                 255

Pro Lys Pro Lys Asp Thr Leu Met Ile Ser Arg Thr Pro Glu Val Thr
                260                 265                 270

Cys Val Val Val Asp Val Ser His Glu Asp Pro Glu Val Lys Phe Asn
                275                 280                 285

Trp Tyr Val Asp Gly Val Glu Val His Asn Ala Lys Thr Lys Pro Arg
    290                 295                 300

Glu Glu Gln Tyr Asn Ser Thr Tyr Arg Val Val Ser Val Leu Thr Val
305                 310                 315                 320

Leu His Gln Asp Trp Leu Asn Gly Lys Glu Tyr Lys Cys Lys Val Ser
                325                 330                 335

Asn Lys Ala Leu Pro Ala Pro Ile Glu Lys Thr Ile Ser Lys Ala Lys
                340                 345                 350

Gly Gln Pro Arg Glu Pro Gln Val Tyr Thr Leu Pro Pro Ser Arg Asp
                355                 360                 365

Glu Leu Thr Lys Asn Gln Val Ser Leu Thr Cys Leu Val Lys Gly Phe
    370                 375                 380

```
Tyr Pro Ser Asp Ile Ala Val Glu Trp Glu Ser Asn Gly Gln Pro Glu
385             390             395             400

Asn Asn Tyr Lys Thr Thr Pro Pro Val Leu Asp Ser Asp Gly Ser Phe
            405             410             415

Phe Leu Tyr Ser Lys Leu Thr Val Asp Lys Ser Arg Trp Gln Gln Gly
            420             425             430

Asn Val Phe Ser Cys Ser Val Met His Glu Ala Leu His Asn His Tyr
            435             440             445

Thr Gln Lys Ser Leu Ser Leu Ser Pro Gly Lys
    450             455
```

<210> 11
<211> 215
<212> PRT
<213> Artificial Sequence

<220>
<223> Light chain Abeta Antibody

<400> 11

EP 2 822 587 B1

```
Asp Ile Val Leu Thr Gln Ser Pro Ala Thr Leu Ser Leu Ser Pro Gly
1               5                   10              15

Glu Arg Ala Thr Leu Ser Cys Arg Ala Ser Gln Ser Val Ser Ser Ser
            20              25              30

Tyr Leu Ala Trp Tyr Gln Gln Lys Pro Gly Gln Ala Pro Arg Leu Leu
            35              40              45

Ile Tyr Gly Ala Ser Ser Arg Ala Thr Gly Val Pro Ala Arg Phe Ser
        50              55              60

Gly Ser Gly Ser Gly Thr Asp Phe Thr Leu Thr Ile Ser Ser Leu Glu
65              70              75              80

Pro Glu Asp Phe Ala Thr Tyr Tyr Cys Leu Gln Ile Tyr Asn Met Pro
                85              90              95

Ile Thr Phe Gly Gln Gly Thr Lys Val Glu Ile Lys Arg Thr Val Ala
            100             105             110

Ala Pro Ser Val Phe Ile Phe Pro Pro Ser Asp Glu Gln Leu Lys Ser
        115             120             125

Gly Thr Ala Ser Val Val Cys Leu Leu Asn Asn Phe Tyr Pro Arg Glu

        130             135             140

Ala Lys Val Gln Trp Lys Val Asp Asn Ala Leu Gln Ser Gly Asn Ser
145             150             155             160

Gln Glu Ser Val Thr Glu Gln Asp Ser Lys Asp Ser Thr Tyr Ser Leu
            165             170             175

Ser Ser Thr Leu Thr Leu Ser Lys Ala Asp Tyr Glu Lys His Lys Val
            180             185             190

Tyr Ala Cys Glu Val Thr His Gln Gly Leu Ser Ser Pro Val Thr Lys
        195             200             205

Ser Phe Asn Arg Gly Glu Cys
210             215
```

**Description**

The present invention relates to a pharmaceutical formulation of an antibody molecule, and/or a mixture of antibody molecules against the amyloid-beta peptide (Abeta).

Antibody molecules, as part of the group of protein pharmaceuticals, are very susceptible to physical and chemical degradation, such as denaturation and aggregation, deamidation, oxidation and hydrolysis. Protein stability is influenced by the characteristics of the protein itself, e.g. the amino acid sequence, and by external influences, such as temperature, solvent pH, excipients, interfaces, or shear rates. So, it is important to define the optimal formulation conditions to protect the protein against degradation reactions during manufacturing, storage and administration. (Manning, M. C., K. Patel, et al. (1989). "Stability of protein pharmaceuticals." Pharm Res 6(11): 903-18., Zheng, J. Y. and L. J. Janis (2005). "Influence of pH, buffer species, and storage temperature on physicochemical stability of a humanized monoclonal antibody LA298." Int)_Pharm.). Administration of antibodies via subcutaneous or intramuscular route requires high protein concentration in the final formulation due to the often required high doses and the limited administration volumes. (Shire, S. J., Z. Shahrokh, et al. (2004). "Challenges in the development of high protein concentration formulations." J Pharm Sci 93(6): 1390-402., Roskos, L. K., C. G. Davis, et al. (2004). "The clinical pharmacology of therapeutic monoclonal antibodies." Drug Development Research 61(3): 108-120.) The large-scale manufacturing of high protein concentration can be achieved by ultrafiltration processes, drying process, such as lyophilisation or spray-drying, and precipitation processes. (Shire, S.1., Z. Shahrokh, et al. (2004). "Challenges in the development of high protein concentration formulations." J Pharm Sci 93(6): 1390-402.).

WO 2008/071394 discloses Abeta antibody compositions.

It is an object of the present invention is to provide a highly concentrated, stable formulation of an Abeta antibody or of mixtures of such antibodies, which allows subcutaneous administration of the antibody to a patient.

The formulation of the present invention shows good stability upon storage for 8 months at 2-8°C and 25°C without formation of visible particles. Shaking and multiple freezing-thawing steps were applied to the liquid formulation to simulate physical stress conditions that potentially occur during manufacturing or transportation of the drug product.

The pharmaceutical formulation of the present invention comprises a poloxamer as surfactant to reduce aggregation of the antibodies and particle formation. The term "poloxamer" as used herein includes a polyoxyethylene-polyoxypropylene triblock copolymer known as poloxamer 188, sold under the trade name PLURONIC® F68 by BASF (Parsippany, N.J.). Other poloxamers which may be utilized in the formulations of the present invention include poloxamer 403 (sold as PLURONIC® P123), poloxamer 407 (sold as PLURONIC® P127), poloxamer 402 (sold as PLURONIC® P122), poloxamer 181 (sold as PLURONIC® L61), poloxamer 401 (sold as PLURONIC® L121), poloxamer 185 (sold as PLURONIC® P65), and poloxamer 338 (sold as PLURONIC® F108).

The present invention provides a stable liquid pharmaceutical antibody formulation comprising:

- 50 mg/ml - 200 mg/ml of an Abeta antibody according to claim 1,

- 0.01 % - 0.1% of a poloxamer, preferably poloxamer 188,

- 5 mM - 50 mM of a buffer,

- 100 mM - 300 mM of a stabilizer,
  at a pH of 4.5 - 7.0

In a particular embodiment of the present invention, the Abeta antibody concentration is about 100 mg/ml - 200 mg/ml, preferably about 150 mg/ml.

In a particular embodiment of the present invention, the poloxamer is present in a concentration of about 0.02% - 0.06%, preferably about 0.04 %.

In a particular embodiment of the present invention, the buffer is a sodium acetate buffer or a Histidine buffer, preferably a Histidine/Histidine-HCl buffer.

In a particular embodiment of the present invention, the buffer has a concentration of about 10 to 30 mM, preferably about 20 mM.

In a particular embodiment of the present invention, the pH of the formulation is about 5 - 6, preferably about 5.5.

In a particular embodiment of the present invention, the stabilizer is selected from sugars and amino acids.

In a particular embodiment of the present invention, the stabilizer is selected from trehalose and arginine.

In a particular embodiment of the present invention, the stabilizer has a concentration of about 100 mM to 300 mM.

In a particular embodiment of the present invention, the stabilizer is threhalose and has a concentration of about 150 mM to 250 mM, preferably about 200 mM.

In a particular embodiment of the present invention, the stabilizer is arginine and has a concentration of about 100 mM to 150 mM, preferably about 135 mM.

In a particular embodiment of the present invention, the Abeta antibody is a monoclonal antibody comprising a heavy chain and a light chain.

In the present invention, the heavy chain of the Abeta antibody comprises a VH domain which comprises:

- a CDR1 comprising the amino acid sequence of Seq. Id. No. 4,
- a CDR2 comprising the amino acid sequence of Seq. Id. No. 5,
- a CDR3 sequence comprising the amino acid sequence of Seq. Id. No. 6.

In the present invention the light chain of the Abeta antibody comprises a VL domain which comprises:

- a CDR1 comprising the amino acid sequence of Seq. Id. No. 7,
- a CDR2 comprising the amino acid sequence of Seq. Id. No. 8,
- a CDR3 sequence comprising the amino acid sequence of Seq. Id. No. 9.

In a particular embodiment of the present invention, the VH domain of the Abeta antibody comprises the amino acid sequence of Seq. Id. No. 2 and the VL domain of the Abeta antibody comprises the amino acid sequence of Seq. Id. No. 3.

In a particular embodiment of the present invention, the heavy chain of the Abeta antibody comprises the amino acid sequence of Seq. Id. No. 10.

In a particular embodiment of the present invention, the light chain of the Abeta antibody comprises the amino acid sequence of Seq. Id. No. 11.

In a particular embodiment of the present invention, the monoclonal Abeta antibody is a mixture of mono-glycosylated Abeta antibodies and double-glycosylated Abeta antibodies, wherein the mono-glycosylated antibody comprises a glycosylated asparagine (Asn) at position 52 of Seq. Id. No. 2 in the VH domain of one antibody binding site and wherein the double-glycosylated antibody comprises a glycosylated asparagine (Asn) at position 52 of Seq. Id. No. 2 in the VH domain of both antibody binding sites and whereby said mixture comprises less than 5% of an antibody being non-glycosylated at position 52 of Seq. Id. No. 2 in the VH domain.

In a particular embodiment the present invention provides the use of the pharmaceutical formulation of the present invention for the subcutaneous administration of the Abeta antibody.

The terms "Abeta antibody" and "an antibody that binds to Abeta" refer to an antibody that is capable of binding Aβ peptide with sufficient affinity such that the antibody is useful as a diagnostic and/or therapeutic agent in targeting Aβ peptide.

It is of note that Aβ has several naturally occurring forms, whereby the human forms are referred to as the above mentioned Aβ39, Aβ40, Aβ41, Aβ42 and Aβ43. The most prominent form, Aβ42, has the amino acid sequence (starting from the N-terminus):

DAEFRHDSGYEVHHQKLVFFAEDVGSNKGAIIGLMVGGVVIA (Seq. Id. No. 1). In Aβ41, Aβ 40, Aβ 39, the C-terminal amino acids A, IA and VIA are missing, respectively. In the Aβ 43 form an additional threonine residue is comprised at the C-terminus of the above depicted sequence (Seq. Id. No. 1).

The term "mono-glycosylated Abeta antibody" relates to an antibody molecule comprising an N-glycosylation at position 52 of Seq. Id. No. 2 in one (VH)-region of an individual antibody molecule; see also figure 1. The term "double-glycosylation Abeta antibody" defines an antibody molecule which is N-glycosylated at position 52 of Seq. Id. No. 2 on both variable regions of the heavy chain" (figure 1). Antibody molecules which lack a N-glycosylation on both heavy chain (VH)-domains are named "non-glycosylated antibodies" (figure 1). The mono-glycosylated antibody, the double-glycosylated antibody and the non-glycosylated antibody may comprise the identical amino acid sequences or different amino acid sequences. The mono-glycosylated antibody and the double-glycosylated antibody are herein referred to as "glycosylated antibody isoforms". A purified antibody molecule characterized in that at least one antigen binding site comprises a glycosylation in the variable region of the heavy chain (VH) is a mono-glycosylated antibody which is free of or to a very low extent associated with an isoform selected from a double-glycosylated antibody and a nonglycosylated antibody, i.e. a "purified mono-glycosylated antibody". A double-glycosylated antibody in context of this invention is free of or to a very low extent associated with an isoform selected from a mono-glycosylated antibody and a non-glycosylated antibody, i.e. a "purified double-glycosylated antibody".

The term "antibody" encompasses the various forms of antibody structures including but not being limited to whole antibodies and antibody fragments. The antibody according to the invention is preferably a humanized antibody, chimeric antibody, or further genetically engineered antibody as long as the characteristic properties according to the invention are retained.

"Antibody fragments" comprise a portion of a full length antibody, preferably the variable domain thereof, or at least the antigen binding site thereof. Examples of antibody fragments include diabodies, single-chain antibody molecules, and multispecific antibodies formed from antibody fragments. scFv antibodies are, e.g. described in Houston, J.S., Methods in Enzymol. 203 (1991) 46-96. In addition, antibody fragments comprise single chain polypeptides having the characteristics of a $V_H$ domain, namely being able to assemble together with a $V_L$ domain, or of a $V_L$ domain binding to A$\beta$, namely being able to assemble together with a $V_H$ domain to a functional antigen binding site and thereby providing the property.

The terms "monoclonal antibody" or "monoclonal antibody composition" as used herein refer to a preparation of antibody molecules of a single amino acid composition.

The term "chimeric antibody" refers to an antibody comprising a variable region, i.e., binding region, from one source or species and at least a portion of a constant region derived from a different source or species, usually prepared by recombinant DNA techniques. Chimeric antibodies comprising a murine variable region and a human constant region are preferred. Other preferred forms of "chimeric antibodies" encompassed by the present invention are those in which the constant region has been modified or changed from that of the original antibody to generate the properties according to the invention, especially in regard to C1q binding and/or Fc receptor (FcR) binding. Such chimeric antibodies are also referred to as "class-switched antibodies.". Chimeric antibodies are the product of expressed immunoglobulin genes comprising DNA segments encoding immunoglobulin variable regions and DNA segments encoding immunoglobulin constant regions. Methods for producing chimeric antibodies involve conventional recombinant DNA and gene transfection techniques are well known in the art. See e.g. Morrison, S.L., et al., Proc. Natl. Acad. Sci. USA 81 (1984) 6851-6855; US Patent Nos. 5,202,238 and 5,204,244.

The term "humanized antibody" refers to antibodies in which the framework or "complementarity determining regions" (CDR) have been modified to comprise the CDR of an immunoglobulin of different specificity as compared to that of the parent immunoglobulin. In a preferred embodiment, a murine CDR is grafted into the framework region of a human antibody to prepare the "humanized antibody." See e.g. Riechmann, L., et al., Nature 332 (1988) 323-327; and Neuberger, M.S., et al., Nature 314 (1985) 268-270. Particularly preferred CDRs correspond to those representing sequences recognizing the antigens noted above for chimeric antibodies. Other forms of "humanized antibodies" encompassed by the present invention are those in which the constant region has been additionally modified or changed from that of the original antibody to generate the properties according to the invention, especially in regard to C1q binding and/or Fc receptor (FcR) binding.

The term "human antibody", as used herein, is intended to include antibodies having variable and constant regions derived from human germ line immunoglobulin sequences. Human antibodies are well-known in the state of the art (van Dijk, M.A., and van de Winkel, J.G., Curr. Opin. Chem. Biol. 5 (2001) 368-374). Human antibodies can also be produced in transgenic animals (e.g., mice) that are capable, upon immunization, of producing a full repertoire or a selection of human antibodies in the absence of endogenous immunoglobulin production. Transfer of the human germ-line immunoglobulin gene array in such germ-line mutant mice will result in the production of human antibodies upon antigen challenge (see, e.g., Jakobovits, A., et al., Proc. Natl. Acad. Sci. USA 90 (1993) 2551-2555; Jakobovits, A., et al., Nature 362 (1993) 255-258; Bruggemann, M., et al., Year Immunol. 7 (1993) 33-40). Human antibodies can also be produced in phage display libraries (Hoogenboom, H.R., and Winter, G., J. Mol. Biol. 227 (1992) 381-388; Marks, J.D., et al., J. Mol. Biol. 222 (1991) 581-597). The techniques of Cole et al. and Boerner et al. are also available for the preparation of human monoclonal antibodies (Cole et al., Monoclonal Antibodies and Cancer Therapy, Alan R. Liss, p. 77 (1985); and Boerner, P., et al., J. Immunol. 147 (1991) 86-95). As already mentioned for chimeric and humanized antibodies according to the invention the term "human antibody" as used herein also comprises such antibodies which are modified in the constant region to generate the properties according to the invention, especially in regard to C1q binding and/or FcR binding, e.g. by "class switching" i.e. change or mutation of Fc parts (e.g. from IgG1 to IgG4 and/or IgG1/IgG4 mutation.).

The term "epitope" includes any polypeptide determinant capable of specific binding to an antibody. In certain embodiments, epitope determinant include chemically active surface groupings of molecules such as amino acids, sugar side chains, phosphoryl, or sulfonyl, and, in certain embodiments, may have specific three dimensional structural characteristics, and or specific charge characteristics. An epitope is a region of an antigen that is bound by an antibody.

The "variable domain" (variable domain of a light chain ($V_L$), variable domain of a heavy chain ($V_H$)) as used herein denotes each of the pair of light and heavy chain domains which are involved directly in binding the antibody to the antigen. The variable light and heavy chain domains have the same general structure and each domain comprises four framework (FR) regions whose sequences are widely conserved, connected by three "hypervariable regions" (or complementary determining regions, CDRs). The framework regions adopt a $\beta$-sheet conformation and the CDRs may form loops connecting the $\beta$-sheet structure. The CDRs in each chain are held in their three-dimensional structure by the framework regions and form together with the CDRs from the other chain the antigen binding site. The antibody's heavy and light chain CDR3 regions play a particularly important role in the binding specificity/affinity of the antibodies according to the invention and therefore provide a further object of the invention.

The term "antigen-binding portion of an antibody" when used herein refer to the amino acid residues of an antibody which are responsible for antigen-binding. The antigen-binding portion of an antibody comprises amino acid residues from the "complementary determining regions" or "CDRs". "Framework" or "FR" regions are those variable domain regions other than the hypervariable region residues as herein defined. Therefore, the light and heavy chain variable domains of an antibody comprise from N- to C-terminus the domains FR1, CDR1, FR2, CDR2, FR3, CDR3, and FR4. Especially, CDR3 of the heavy chain is the region which contributes most to antigen binding and defines the antibody's properties. CDR and FR regions are determined according to the standard definition of Kabat et al., Sequences of Proteins of Immunological Interest, 5th ed., Public Health Service, National Institutes of Health, Bethesda, MD (1991) and/or those residues from a "hypervariable loop".

The term "stabilizer" denotes a pharmaceutical acceptable excipient, which protects the active pharmaceutical ingredient and / or the formulation from chemical and / or physical degradation during manufacturing, storage and application. Chemical and physical degradation pathways of protein pharmaceuticals are reviewed by Cleland, J. L., M. F. Powell, et al. (1993). "The development of stable protein formulations: a close look at protein aggregation, deamidation, and oxidation." Crit Rev Ther Drug Carrier Syst 10(4): 307-77, Wang, W. (1999). "Instability, stabilization, and formulation of liquid protein pharmaceuticals." Int J Pharm 185(2): 129-88., Wang, W. (2000). "Lyophilization and development of solid protein pharmaceuticals." Int J Pharm 203(1-2): 1-60. and Chi, E. Y.,. S. Krishnan, et ai. (2003). "Physical stability of proteins in aqueous solution: mechanism and driving forces in nonnative protein aggregation." Pharm Res 20(9): 1325-36. Stabilizers include but are not limited to sugars, amino acids, polyols, surfactants, antioxidants, preservatives, cyclodextrines, polyethylenglycols, e.g. PEG 3000, 3350, 4000, 6000, albumin, e.g. human serum albumin (HSA), bovines serum albumin (BSA), salts, e.g. sodium chloride, magnesium chloride, calcium chloride, chelators, e.g. EDTA as hereafter defined. As mentioned hereinabove, stabilizers can be present in the formulation in an amount of about 10 to about 500 mM, preferably in an amount of about 10 to about 300 mM and more preferably in an amount of about 100 mM to about 300 mM.

A "stable liquid pharmaceutical antibody formulation" is a liquid antibody formulation with no significant changes observed at a refrigerated temperature (2-8 °C) for at least 12 months, particularly 2 years, and more particularly 3 years. The criteria for stability are the following: no more than 10%, particularly 5%, of antibody monomer is degraded as measured by size exclusion chromatography (SEC-HPLC). Furthermore, the solution is colorless or clear to slightly opalescent by visual analysis. The protein concentration of the formulation has no more than +/-10% change. No more than 10%, particularly 5% of aggregation is formed. The stability is measured by methods known in the art such UV spectroscopy, size exclusion chromatography (SEC-HPLC), Ion-Exchange Chromatography (IE-HPLC), turbidimetry and visual inspection.

Recombinant Methods and Compositions

Antibodies may be produced using recombinant methods and compositions, e.g., as described in U.S. Patent No. 4,816,567. In one embodiment, isolated nucleic acid encoding an anti-[[PRO]] antibody described herein is provided. Such nucleic acid may encode an amino acid sequence comprising the VL and/or an amino acid sequence comprising the VH of the antibody (e.g., the light and/or heavy chains of the antibody). In a further embodiment, one or more vectors (e.g., expression vectors) comprising such nucleic acid are provided. In a further embodiment, a host cell comprising such nucleic acid is provided. In one such embodiment, a host cell comprises (e.g., has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In one embodiment, the host cell is eukaryotic, e.g. a Chinese Hamster Ovary (CHO) cell or lymphoid cell (e.g., Y0, NS0, Sp20 cell). In one embodiment, a method of making an anti-[[PRO]] antibody is provided, wherein the method comprises culturing a host cell comprising a nucleic acid encoding the antibody, as provided above, under conditions suitable for expression of the antibody, and optionally recovering the antibody from the host cell (or host cell culture medium).

For recombinant production of an anti-Abeta antibody, nucleic acid encoding an antibody, e.g., as described above, is isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, see, e.g., U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments in *E. coli.)* After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, Nat. Biotech. 22:1409-1414 (2004), and Li et al., Nat. Biotech. 24:210-215 (2006).

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. *See,* e.g., US Patent Nos. 5,959,177, 6,040,498, 6,420,548, 7,125,978, and 6,417,429 (describing PLANTIBODIES™ technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham et al., J. Gen Virol. 36:59 (1977)); baby hamster kidney cells (BHK); mouse sertoli cells (TM4 cells as described, e.g., in Mather, Biol. Reprod. 23:243-251 (1980)); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, e.g., in Mather et al., Annals N.Y. Acad. Sci. 383:44-68 (1982); MRC 5 cells; and FS4 cells. Other useful mammalian host cell lines include Chinese hamster ovary (CHO) cells, including DHFR$^-$ CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, see, e.g., Yazaki and Wu, Methods in Molecular Biology, Vol. 248 (B.K.C. Lo, ed., Humana Press, Totowa, NJ), pp. 255-268 (2003).

## Examples

Liquid drug product formulations for subcutaneous administration according to the invention were developed as follows.

Example 1: Preparation of liquid formulations

The following Abeta liquid formulations were prepared at a protein concentration of 150 mg/ml:

| Code | Buffer | Surfactant | Excipient |
|------|--------|-----------|-----------|
| F1 | 20 mM Sodium Acetate pH 5.5 | 0.02% Polysorbate 20 | 200 mM Trehalose |
| F2 | | 0.02% Polysorbate 20 | 210 mM Sorbitol |
| F3 | | 0.02% Polysorbate 20 | 135 mM Arginine |
| F4 | | 0.02% Polysorbate 80 | 200 mM Trehalose |
| F5 | | 0.02% Polysorbate 80 | 210 mM Sorbitol |
| F6 | | 0.02% Polysorbate 80 | 135 mM Arginine |
| F7 | | 0.04% Poloxamer 188 | 200 mM Trehalose |
| F8 | | 0.04% Poloxamer 188 | 135 mM Arginine |
| F9 | 20mM Histidine/Histidine-HCl pH 5.5 | 0.02% Polysorbate 20 | 200 mM Trehalose |
| F10 | | 0.02% Polysorbate 20 | 210 mM Sorbitol |
| F11 | | 0.02% Polysorbate 20 | 135 mM Arginine |
| F12 | | 0.02% Polysorbate 80 | 200 mM Trehalose |
| F13 | | 0.02% Polysorbate 80 | 210 mM Sorbitol |
| F14 | | 0.02% Polysorbate 80 | 135 mM Arginine |
| F15 | | 0.04% Poloxamer 188 | 200 mM Trehalose |
| F16 | | 0.04% Poloxamer 188 | 135 mM Arginine |

Abeta antibody prepared and obtained as described in WO2007/068429 was provided at a concentration of approx. 50-60 mg/mL in a 10 mM histidine buffer at a pH of approx. 5.5. The Abeta antibody used in the examples comprises the CDRs, VH domain, VL domain, heavy chain and light chain specified in the Sequence Listing of the present application (Seq. Id. No. 2 - 11).

For the preparation of the liquid formulations Abeta was buffer-exchanged against a diafiltration buffer containing the anticipated buffer composition and concentrated by ultrafiltration to an antibody concentration of approx. 200 mg/mL. After completion of the ultrafiltration operation, the excipients (e.g. trehalose) were added as stock solutions to the antibody solution. The surfactant was then added as a 50 to 125-fold stock solution. Finally the protein concentration was adjusted with a buffer to the final Abeta concentration of approx. 150 mg/mL.

All formulations were sterile-filtered through 0.22 $\mu$m low protein binding filters and aseptically filled into sterile 6 mL glass vials closed with ETFE (Copolymer of ethylene and tetrafluoroethylene)-coated rubber stoppers and alucrimp caps. The fill volume was approx. 2.4 mL. These formulations were stored at different climate conditions (5°C, 25°C and 40°C) for different intervals of time and stressed by shaking (1 week at a shaking frequency of 200 min-1 at 5°C and 25°C) and freeze-thaw stress methods (five cycles at -80°C/+5°C). The samples were analyzed before and after applying the stress tests as well as after storage by the following analytical methods:

- UV spectroscopy

- Size Exclusion Chromatography (SEC)

- Ion exchange chromatography (IEC)

- Clarity and opalescence of the solution

- Visual inspection

UV spectroscopy, used for determination of protein content, was performed on a Perkin Elmer $\lambda$35 UV spectrophotometer in a wavelength range from 240 nm to 400 nm. Neat protein samples were diluted to approx. 0.5 mg/mL with the corresponding formulation buffer. The protein concentration was calculated according to equation 1.

$$\text{Equation 1:} \quad \text{Protein content} = \frac{A(280) - A(320) \times dil.factor}{\varepsilon \left\langle {cm^2}/{mg} \right\rangle \times d \langle cm \rangle}$$

The UV light absorption at 280 nm was corrected for light scattering at 320 nm and multiplied with the dilution factor, which was determined from the weighed masses and densities of the neat sample and the dilution buffer. The numerator was divided by the product of the cuvette's path length d and the extinction coefficient $\varepsilon$.

Size Exclusion Chromatography (SEC) was used to detect soluble high molecular weight species (aggregates) and low molecular weight hydrolysis products (LMW) in the formulations. The method was performed on a Waters Alliance 2695 HPLC instrument with a Waters W2487 Dual Absorbance Detector and equipped with a TosoHaas TSK-Gel G3000SWXL column. Intact monomer, aggregates and hydrolysis products were separated by an isocratic elution profile, using 0.2M K2HPO4 / 0.25M KCL, pH 7.0 as mobile phase, and were detected at a wavelength of 280 nm.

Ion Exchange Chromatography (IEC) was performed to detect chemical degradation products altering the net charge of Abeta in the formulations. The method used a Waters Alliance 2695 HPLC instrument with a Waters W2487 Dual Absorbance Detector and equipped (detection wavelength 280nm) and a Mono S TM 5/50GL column (Amersham Biosciences). 50 mM malonic acid/malonate pH 5.3 and 1M Na-acetate in Mobile Phase A pH 5.3 used as mobile phases A and B, respectively, with a flow rate of 1.0 mL/min.

Gradient program:

| min | Mobile Phase A | Mobile Phase B |
|-----|----------------|----------------|
| 0 | 100 | 0 |
| 1 | 100 | 0 |
| 20 | 48 | 52 |

| | | |
|---|---|---|
| 22 | 48 | 52 |
| 24 | 0 | 100 |
| 25 | 0 | 100 |
| 26 | 100 | 0 |
| 30 | 100 | 0 |

Clarity and the degree of opalescence were measured as Formazine Turbidity Units (FTU) by the method of nephelometry. The neat sample was transferred into a 11 mm diameter clear-glass tube and placed into a HACH 2100AN turbidimeter.

Samples were inspected for the presence of visible particles by using a Seidenader V90-T visual inspection instrument.

**Compositions and stability data of liquid Abeta drug product formulations according to this invention**

**F1** is a liquid formulation with the composition 150 mg/mL Abeta, 20 mM sodium acetate, 200 mM trehalose, 0.02% polysorbate 20, at pH 5.5

| Storage condition | Storage Time | Protein conc. (mg/mL) | Size Exclusion-HPLC | | | Ion Exchange-HPLC | | Turbidity (FTU) | Visible particles |
|---|---|---|---|---|---|---|---|---|---|
| | | | HMW (%) | Monomer (%) | LMW (%) | Peak 1 (%) | Peak 2 (%) | | |
| - | Initial | 151.8 | 2.3 | 97.0 | 0.7 | 23.7 | 23.7 | 3.3 | Practically free from particles |
| Shaking 5°C | 1 week | - | 2.3 | 96.7 | 1.0 | - | - | 3.5 | Practically free from particles |
| Shaking 25°C | 1 week | 150.0 | 2.3 | 96.9 | 0.9 | - | - | 3.3 | Practically free from particles |
| Freeze/thaw | 5 cycles | - | 2.4 | 96.8 | 0.8 | - | - | 3.8 | Practically free from particles |
| 5°C | 8 months | - | 2.8 | 97.1 | 0.1 | 23.2 | 23.2 | 6.5 | With many particles |
| 25°C | 8 months | - | 3.5 | 94.5 | 2.0 | 15.8 | 15.8 | 6.5 | With many particles |
| 40°C | 8 months | 152.0 | 9.5 | 79.6 | 11.0 | 3.4 | 3.4 | 7.8 | With many particles |

**F2** is a liquid formulation with the composition 150 mg/mL Abeta, 20 mM sodium acetate, 210 mM sorbitol, 0.02% polysorbate 20, at pH 5.5

| Storage condition | Storage Time | Protein conc. (mg/mL) | Size Exclusion-HPLC | | | Ion Exchange-HPLC | | Turbidity (FTU) | Visible particles |
|---|---|---|---|---|---|---|---|---|---|
| | | | HMW (%) | Monomer (%) | LMW (%) | Peak 1 (%) | Peak 2 (%) | | |
| - | Initial | 153.2 | 2.2 | 97.2 | 0.7 | 45.3 | 23.7 | 3.6 | Practically free from particles |
| Shaking 5°C | 1 week | - | 2.3 | 96.9 | 0.8 | - | - | 3.6 | Practically free from particles |
| Shaking 25°C | 1 week | 152.1 | 2.3 | 97.1 | 0.8 | - | - | 3.9 | Practically free from particles |
| Freeze/thaw | 5 cycles | - | 2.2 | 96.9 | 0.8 | - | - | 4.2 | Practically free from particles |
| 5°C | 8 months | - | 2.7 | 97.2 | 0.1 | 47.7 | 23.1 | 7.8 | With many particles |
| 25°C | 8 months | - | 3.6 | 94.4 | 2.1 | 61.0 | 15.6 | 7.0 | With many particles |
| 40°C | 8 months | 151.8 | 10.3 | 78.6 | 11.1 | 67.9 | 3.5 | 7.5 | With many particles |

**F3** is a liquid formulation with the composition 150 mg/mL Abeta, 20 mM sodium acetate, 135 mM Arginine, 0.02% polysorbate 20, at pH 5.5

| Storage condition | Storage Time | Protein conc. (mg/mL) | Size Exclusion-HPLC | | | Ion Exchange-HPLC | | Turbidity (FTU) | Visible particles |
|---|---|---|---|---|---|---|---|---|---|
| | | | HMW (%) | Monomer (%) | LMW (%) | Peak 1 (%) | Peak 2 (%) | | |
| - | Initial | 154.5 | 1.8 | 97.5 | 0.7 | 45.8 | 23.8 | 11.4 | Practically free from particles |
| Shaking 5°C | 1 week | - | 1.9 | 97.3 | 0.9 | - | - | 11.9 | Practically free from particles |
| Shaking 25°C | 1 week | 153.1 | 1.9 | 97.4 | 0.8 | - | - | 11.3 | Practically free from particles |
| Freeze/thaw | 5 cycles | - | 1.8 | 97.3 | 0.8 | - | - | 10.9 | Practically free from particles |
| 5°C | 8 months | - | 2.3 | 97.6 | 0.1 | 23.4 | 13.3 | 13.3 | With many particles |
| 25°C | 8 months | - | 2.7 | 95.1 | 2.2 | 17.6 | 13.4 | 13.4 | With many particles |
| 40°C | 8 months | 152.1 | 8.6 | 78.5 | 12.9 | 4.8 | 12.1 | 12.1 | With many particles |

**F4** is a liquid formulation with the composition 150 mg/mL Abeta, 20 mM sodium acetate, 200 mM trehalose, 0.02% polysorbate 80, at pH 5.5

| Storage condition | Storage Time | Protein conc. (mg/mL) | Size Exclusion-HPLC | | | Ion Exchange-HPLC | | Turbidity (FTU) | Visible particles |
|---|---|---|---|---|---|---|---|---|---|
| | | | HMW (%) | Monomer (%) | LMW (%) | Peak 1 (%) | Peak 2 (%) | | |
| - | Initial | 152.3 | 2.2 | 97.1 | 0.7 | 45.3 | 23.8 | 3.8 | Practically free from particles |
| Shaking 5°C | 1 week | - | 2.2 | 97.0 | 0.8 | - | - | 3.4 | Practically free from particles |
| Shaking 25°C | 1 week | 151.4 | 2.2 | 97.1 | 0.7 | - | - | 3.8 | Practically free from particles |
| Freeze/thaw | 5 cycles | - | 2.4 | 96.8 | 0.8 | - | - | 3.5 | Practically free from particles |
| 5°C | 8 months | - | 2.8 | 97.1 | 0.1 | 47.7 | 23.2 | 3.6 | Practically free from particles |
| 25°C | 8 months | - | 3.5 | 94.5 | 2.0 | 60.7 | 15.7 | 5.2 | With many particles |
| 40°C | 8 months | 149.3 | 9.8 | 79.0 | 11.2 | 68.0 | 3.5 | 7.2 | With many particles |

**F5** is a liquid formulation with the composition 150 mg/mL Abeta, 20 mM sodium acetate, 210 mM sorbitol, 0.02% polysorbate 80, at pH 5.5

| Storage condition | Storage Time | Protein conc. (mg/mL) | Size Exclusion-HPLC | | | Ion Exchange-HPLC | | Turbidity (FTU) | Visible particles |
|---|---|---|---|---|---|---|---|---|---|
| | | | HMW (%) | Monomer (%) | LMW (%) | Peak 1 (%) | Peak 2 (%) | | |
| - | Initial | 153.4 | 2.0 | 97.4 | 0.6 | 45.9 | 23.8 | 4.2 | Practically free from particles |
| Shaking 5°C | 1 week | - | 2.1 | 97.0 | 0.9 | - | - | 4.2 | Practically free from particles |
| Shaking 25°C | 1 week | 153.1 | 2.1 | 97.1 | 0.8 | - | - | 4.0 | Practically free from particles |
| Freeze/thaw | 5 cycles | - | 2.3 | 97.0 | 0.8 | - | - | 4.0 | Practically free from particles |
| 5°C | 8 months | - | 2.8 | 97.2 | 0.1 | 47.7 | 23.2 | 4.2 | Practically free from particles |
| 25°C | 8 months | - | 3.5 | 94.5 | 2.0 | 60.9 | 15.6 | 5.6 | With many particles |
| 40°C | 8 months | 151.1 | 10.2 | 78.7 | 11.1 | 67.9 | 3.5 | 9.4 | With many particles |

**F6** is a liquid formulation with the composition 150 mg/mL Abeta, 20 mM sodium acetate, 135 mM Arginine, 0.02% polysorbate 80, at pH 5.5

| Storage condition | Storage Time | Protein conc. (mg/mL) | Size Exclusion-HPLC | | | Ion Exchange-HPLC | | Turbidity (FTU) | Visible particles |
|---|---|---|---|---|---|---|---|---|---|
| | | | HMW (%) | Monomer (%) | LMW (%) | Peak 1 (%) | Peak 2 (%) | | |
| - | Initial | 155.2 | 1.7 | 97.7 | 0.6 | 46.0 | 23.8 | 11.1 | Practically free from particles |
| Shaking 5°C | 1 week | - | 1.8 | 97.5 | 0.8 | - | - | 10.9 | Practically free from particles |
| Shaking 25°C | 1 week | 152.0 | 1.7 | 97.6 | 0.7 | - | - | 11.1 | Practically free from particles |
| Freeze/thaw | 5 cycles | - | 1.9 | 97.3 | 0.8 | - | - | 11.0 | Practically free from particles |
| 5°C | 8 months | - | 2.3 | 97.7 | 0.1 | 46.7 | 23.3 | 10.6 | With many particles |
| 25°C | 8 months | - | 2.7 | 95.0 | 2.2 | 57.2 | 17.6 | 11.1 | Practically free from particles |
| 40°C | 8 months | 152.9 | 9.1 | 77.9 | 13.1 | 65.6 | 4.9 | 12.0 | With many particles |

**F7** is a liquid formulation with the composition 150 mg/mL Abeta, 20 mM sodium acetate, 200 mM trehalose, 0.04% poloxamer 188, at pH 5.5

| Storage condition | Storage Time | Protein conc. (mg/mL) | Size Exclusion-HPLC | | | Ion Exchange-HPLC | | Turbidity (FTU) | Visible particles |
|---|---|---|---|---|---|---|---|---|---|
| | | | HMW (%) | Monomer (%) | LMW (%) | Peak 1 (%) | Peak 2 (%) | | |
| - | Initial | 149.5 | 2.0 | 97.9 | 0.2 | 49.9 | 23.9 | 3.88 | Practically free from particles |
| Shaking 5°C | 1 week | - | 2.1 | 97.8 | 0.1 | - | - | 3.50 | Practically free from particles |
| Shaking 25°C | 1 week | 147.75 | 2.2 | 97.6 | 0.2 | - | - | 3.32 | Practically free from particles |
| Freeze/thaw | 5 cycles | - | 2.1 | 97.8 | 0.1 | - | - | 3.43 | Practically free from particles |
| 5°C | 8 months | - | 2.4 | 97.2 | 0.4 | 53.1 | 23.8 | 13.3 | Practically free from particles |
| 25°C | 8 months | - | 3.3 | 94.0 | 2.8 | 62.3 | 17.1 | 4.14 | Practically free from particles |
| 40°C | 8 months | 151.20 | 10.3 | 77.0 | 12.7 | 69.0 | 2.4 | 4.70 | Practically free from particles |

**F8** is a liquid formulation with the composition 150 mg/mL Abeta, 20 mM sodium acetate, 135 mM Arginine, 0.04% poloxamer 188, at pH 5.5

| Storage condition | Storage Time | Protein conc. (mg/mL) | Size Exclusion-HPLC | | | Ion Exchange-HPLC | | Turbidity (FTU) | Visible particles |
|---|---|---|---|---|---|---|---|---|---|
| | | | HMW (%) | Monomer (%) | LMW (%) | Peak 1 (%) | Peak 2 (%) | | |
| - | Initial | 148.90 | 1.7 | 98.1 | 0.1 | 50.5 | 23.9 | 12.9 | Practically free from particles |
| Shaking 5°C | 1 week | - | 1.8 | 98.0 | 0.1 | - | - | 12.6 | Practically free from particles |
| Shaking 25°C | 1 week | 144.89 | 1.9 | 98.0 | 0.2 | - | - | 12.2 | Practically free from particles |
| Freeze/thaw | 5 cycles | - | 1.8 | 98.0 | 0.1 | - | - | 13.30 | Practically free from particles |
| 5°C | 8 months | - | 2.0 | 97.6 | 0.4 | 51.6 | 23.9 | 15.1 | Practically free from particles |
| 25°C | 8 months | - | 2.6 | 94.4 | 3.0 | 59.2 | 19.0 | 13.1 | Practically free from particles |
| 40°C | 8 months | 150.32 | 9.6 | 75.3 | 15.1 | 66.0 | 3.9 | 17.1 | Practically free from particles |

**F9** is a liquid formulation with the composition 150 mg/mL Abeta, 20 mM histidine, 200 mM trehalose, 0.02% polysorbate 20, at pH 5.5

| Storage condition | Storage Time | Protein conc. (mg/mL) | Size Exclusion-HPLC | | | Ion Exchange-HPLC | | Turbidity (FTU) | Visible particles |
|---|---|---|---|---|---|---|---|---|---|
| | | | HMW (%) | Monomer (%) | LMW (%) | Peak 1 (%) | Peak 2 (%) | | |
| - | Initial | 150.7 | 1.7 | 97.7 | 0.7 | 44.9 | 23.7 | 4.4 | Practically free from particles |
| Shaking 5°C | 1 week | - | 1.7 | 97.6 | 0.7 | - | - | 4.0 | Practically free from particles |
| Shaking 25°C | 1 week | 149.0 | 1.7 | 97.5 | 0.7 | - | - | 4.7 | Practically free from particles |
| Freeze/thaw | 5 cycles | - | 1.9 | 97.3 | 0.8 | - | - | 4.4 | Practically free from particles |
| 5°C | 8 months | - | 2.2 | 97.7 | 0.1 | 47.7 | 23.3 | 7.4 | With many particles |
| 25°C | 8 months | - | 2.9 | 95.0 | 2.1 | 58.3 | 17.9 | 7.7 | With many particles |
| 40°C | 8 months | 150.2 | 9.0 | 78.3 | 12.8 | 66.7 | 5.1 | 7.4 | With many particles |

**F10** is a liquid formulation with the composition 150 mg/mL Abeta, 20 mM histidine, 210 mM sorbitol, 0.02% polysorbate 20, at pH 5.5

| Storage condition | Storage Time | Protein conc. (mg/mL) | Size Exclusion-HPLC | | | Ion Exchange-HPLC | | Turbidity (FTU) | Visible particles |
|---|---|---|---|---|---|---|---|---|---|
| | | | HMW (%) | Monomer (%) | LMW (%) | Peak 1 (%) | Peak 2 (%) | | |
| - | Initial | 152.6 | 1.7 | 97.7 | 0.7 | 46.3 | 23.7 | 4.82 | Practically free from particles |
| Shaking 5°C | 1 week | - | 1.7 | 97.6 | 0.7 | - | - | 4.80 | Practically free from particles |
| Shaking 25°C | 1 week | 151.4 | 1.7 | 97.6 | 0.7 | - | - | 4.45 | Practically free from particles |
| Freeze/thaw | 5 cycles | - | 1.9 | 97.4 | 0.8 | - | - | 4.67 | Practically free from particles |
| 5°C | 8 months | - | 2.2 | 97.7 | 0.1 | 47.5 | 23.3 | 7.38 | With many particles |
| 25°C | 8 months | - | 2.9 | 95.1 | 2.0 | 58.5 | 17.8 | 7.69 | With many particles |
| 40°C | 8 months | 152.0 | 8.7 | 78.4 | 12.9 | 66.5 | 5.1 | 7.48 | With many particles |

**F11** is a liquid formulation with the composition 150 mg/mL Abeta, 20 mM histidine, 135 mM Arginine, 0.02% polysorbate 20, at pH 5.5

| Storage condition | Storage Time | Protein conc. (mg/mL) | Size Exclusion-HPLC | | | Ion Exchange-HPLC | | Turbidity (FTU) | Visible particles |
|---|---|---|---|---|---|---|---|---|---|
| | | | HMW (%) | Monomer (%) | LMW (%) | Peak 1 (%) | Peak 2 (%) | | |
| - | Initial | 154.1 | 1.5 | 97.9 | 0.6 | 45.2 | 23.8 | 11.5 | Practically free from particles |
| Shaking 5°C | 1 week | - | 1.6 | 97.7 | 0.7 | - | - | 11.4 | Practically free from particles |
| Shaking 25°C | 1 week | 152.0 | 1.6 | 97.7 | 0.7 | - | - | 10.8 | Practically free from particles |
| Freeze/thaw | 5 cycles | - | 1.7 | 97.6 | 0.8 | - | - | 11.4 | Practically free from particles |
| 5°C | 8 months | - | 2.0 | 97.9 | 0.1 | 46.6 | 23.4 | 13.1 | With many particles |
| 25°C | 8 months | - | 2.4 | 95.4 | 2.2 | 55.8 | 18.7 | 15.0 | With many particles |
| 40°C | 8 months | 153.5 | 8.0 | 77.7 | 14.3 | 66.5 | 5.1 | 11.8 | With many particles |

**F12** is a liquid formulation with the composition 150 mg/mL Abeta, 20 mM histidine, 200 mM trehalose, 0.02% polysorbate 80, at pH 5.5

| Storage condition | Storage Time | Protein conc. (mg/mL) | Size Exclusion-HPLC | | | Ion Exchange-HPLC | | Turbidity (FTU) | Visible particles |
|---|---|---|---|---|---|---|---|---|---|
| | | | HMW (%) | Monomer (%) | LMW (%) | Peak 1 (%) | Peak 2 (%) | | |
| - | Initial | 151.0 | 1.7 | 97.6 | 0.7 | 45.5 | 23.7 | 4.53 | Practically free from particles |
| Shaking 5°C | 1 week | - | 1.7 | 97.6 | 0.7 | - | - | 4.40 | Practically free from particles |
| Shaking 25°C | 1 week | 151.3 | 1.7 | 97.5 | 0.8 | - | - | 4.20 | Practically free from particles |
| Freeze/thaw | 5 cycles | - | 2.0 | 97.2 | 0.8 | - | - | 4.41 | Practically free from particles |
| 5°C | 8 months | - | 2.2 | 97.7 | 0.1 | 47.7 | 23.3 | 4.43 | With many particles |
| 25°C | 8 months | - | 2.9 | 94.9 | 2.1 | 58.3 | 17.9 | 6.24 | With many particles |
| 40°C | 8 months | 150.9 | 9.1 | 78.2 | 12.8 | 66.8 | 5.1 | 9.88 | With many particles |

**F13** is a liquid formulation with the composition 150 mg/mL Abeta, 20 mM histidine, 210 mM sorbitol, 0.02% polysorbate 80, at pH 5.5

| Storage condition | Storage Time | Protein conc. (mg/mL) | Size Exclusion-HPLC | | | Ion Exchange-HPLC | | Turbidity (FTU) | Visible particles |
|---|---|---|---|---|---|---|---|---|---|
| | | | HMW (%) | Monomer (%) | LMW (%) | Peak 1 (%) | Peak 2 (%) | | |
| - | Initial | 153.2 | 1.7 | 97.6 | 0.7 | 46.1 | 23.7 | 4.68 | Practically free from particles |
| Shaking 5°C | 1 week | - | 1.7 | 97.6 | 0.7 | - | - | 4.47 | Practically free from particles |
| Shaking 25°C | 1 week | 152.2 | 1.8 | 97.5 | 0.7 | - | - | 4.73 | Practically free from particles |
| Freeze/thaw | 5 cycles | - | 1.9 | 97.3 | 0.8 | - | - | 4.54 | Practically free from particles |
| 5°C | 8 months | - | 2.2 | 97.7 | 0.1 | 47.5 | 23.3 | 5.24 | With many particles |
| 25°C | 8 months | - | 2.9 | 95.0 | 2.1 | 58.5 | 17.8 | 6.33 | With many particles |
| 40°C | 8 months | 152.1 | 8.8 | 78.2 | 13.0 | 66.6 | 5.2 | 10.8 | With many particles |

**F14** is a liquid formulation with the composition 150 mg/mL Abeta, 20 mM histidine, 135 mM Arginine, 0.02% polysorbate 80, at pH 5.5

| Storage condition | Storage Time | Protein conc. (mg/mL) | Size Exclusion-HPLC | | | Ion Exchange-HPLC | | Turbidity (FTU) | Visible particles |
|---|---|---|---|---|---|---|---|---|---|
| | | | HMW (%) | Monomer (%) | LMW (%) | Peak 1 (%) | Peak 2 (%) | | |
| - | Initial | 154.7 | 1.6 | 97.7 | 0.8 | 45.7 | 23.8 | 11.3 | Practically free from particles |
| Shaking 5°C | 1 week | - | 1.6 | 97.7 | 0.7 | - | - | 10.9 | Practically free from particles |
| Shaking 25°C | 1 week | 153.0 | 1.6 | 97.6 | 0.8 | - | - | 11.8 | Practically free from particles |
| Freeze/thaw | 5 cycles | - | 1.8 | 97.5 | 0.8 | - | - | 10.9 | Practically free from particles |
| 5°C | 8 months | - | 2.0 | 97.9 | 0.1 | 46.7 | 23.3 | 11.1 | With many particles |
| 25°C | 8 months | - | 2.5 | 95.3 | 2.3 | 55.9 | 18.7 | 11.4 | With many particles |
| 40°C | 8 months | 155.1 | 8.5 | 76.7 | 14.7 | 64.2 | 6.6 | 13.3 | With many particles |

**F15** is a liquid formulation with the composition 150 mg/mL Abeta, 20 mM histidine, 200 mM trehalose, 0.04% poloxamer 188, at pH 5.5

| Storage condition | Storage Time | Protein conc. (mg/mL) | Size Exclusion-HPLC | | | Ion Exchange-HPLC | | Turbidity (FTU) | Visible particles |
|---|---|---|---|---|---|---|---|---|---|
| | | | HMW (%) | Monomer (%) | LMW (%) | Peak 1 (%) | Peak 2 (%) | | |
| - | Initial | 151.1 | 1.8 | 98.1 | 0.2 | 50.5 | 23.9 | 4.94 | Practically free from particles |
| Shaking 5°C | 1 week | - | 1.9 | 98.0 | 0.1 | - | - | 4.42 | Practically free from particles |
| Shaking 25°C | 1 week | 150.7 | 2.0 | 97.8 | 0.2 | - | - | 4.06 | Practically free from particles |
| Freeze/thaw | 5 cycles | - | 1.9 | 98.0 | 0.1 | - | - | 4.25 | Practically free from particles |
| 5°C | 8 months | - | 2.1 | 97.5 | 0.4 | 51.9 | 23.9 | n.d. | Practically free from particles |
| 25°C | 8 months | - | 2.9 | 94.2 | 2.9 | 59.6 | 19.6 | 5.40 | Practically free from particles |
| 40°C | 8 months | 152.3 | 9.7 | 74.4 | 15.9 | 66.5 | 5.0 | 6.36 | Practically free from particles |

**F16** is a liquid formulation with the composition 150 mg/mL Abeta, 20 mM histidine, 135 mM Arginine, 0.04% poloxamer 188, at pH 5.5

| Storage condition | Storage Time | Protein conc. (mg/mL) | Size Exclusion-HPLC | | | Ion Exchange-HPLC | | Turbidity (FTU) | Visible particles |
|---|---|---|---|---|---|---|---|---|---|
| | | | HMW (%) | Monomer (%) | LMW (%) | Peak 1 (%) | Peak 2 (%) | | |
| - | Initial | 151.8 | 1.6 | 98.2 | 0.2 | 50.4 | 23.9 | 13.0 | Practically free from particles |
| Shaking 5°C | 1 week | - | 1.7 | 98.1 | 0.1 | - | - | 12.5 | Practically free from particles |
| Shaking 25°C | 1 week | 147.9 | 1.8 | 98.1 | 0.2 | - | - | 12.7 | Practically free from particles |
| Freeze/thaw | 5 cycles | - | 1.7 | 98.1 | 0.1 | - | - | 12.8 | Practically free from particles |
| 5°C | 8 months | - | 1.9 | 97.7 | 0.4 | 51.8 | 23.9 | 16.8 | Practically free from particles |
| 25°C | 8 months | - | 2.3 | 94.6 | 3.1 | 57.5 | 20.6 | 12.9 | Practically free from particles |
| 40°C | 8 months | 152.2 | 8.9 | 72.5 | 18.6 | 62.6 | 7.3 | 14.2 | Practically free from particles |

The stability data presented above show that all of the polysorbate 20 and polysorbate 80 containing formulations are developing visible particles after 8 months storage at 5°C, 25°C or 40°C. On the other hand, the poloxamer containing formulations are practically free from visible particles after storage for 8 months at 5°C, 25°C and 40°C. Therefore poloxamer is able to prevent the formation of visible particles in Abeta antibody formulations.

**Amino acid sequences disclosed in the application**

| Amino acid sequence | Seq. Id. No. |
|---|---|
| Abeta peptide Aβ | 1 |
| VH domain of Abeta antibody | 2 |
| VL domain of Abeta antibody | 3 |
| CDR1 of VH domain of Abeta antibody | 4 |
| CDR2 of VH domain of Abeta antibody | 5 |
| CDR3 of VH domain of Abeta antibody | 6 |
| CDR1 of VL domain of Abeta antibody | 7 |
| CDR2 of VL domain of Abeta antibody | 8 |
| CDR3 of VL domain of Abeta antibody | 9 |
| Heavy chain Abeta antibody | 10 |
| Light chain Abeta antibody | 11 |

## Claims

1. A stable liquid pharmaceutical antibody formulation comprising:

- 50 mg/ml - 200 mg/ml of an Abeta antibody,
- 0.01 % - 0.1% of a poloxamer, preferably poloxamer 188,
- 5 mM - 50 mM of a buffer,
- 100 mM - 300 mM of a stabilizer,

at a pH of 4.5 - 7.0,
wherein the Abeta antibody is a monoclonal antibody comprising a heavy chain and a light chain, wherein the heavy chain of the Abeta antibody comprises a VH domain which comprises:

- a CDR1 comprising the amino acid sequence of Seq. Id. No. 4,
- a CDR2 comprising the amino acid sequence of Seq. Id. No. 5,
- a CDR3 sequence comprising the amino acid sequence of Seq. Id. No. 6, and

the light chain of the Abeta antibody comprises a VL domain which comprises:

- a CDR1 comprising the amino acid sequence of Seq. Id. No. 7,
- a CDR2 comprising the amino acid sequence of Seq. Id. No. 8,
- a CDR3 sequence comprising the amino acid sequence of Seq. Id. No. 9.

2. The pharmaceutical formulation of claim 1, wherein the Abeta antibody concentration is 100 mg/ml - 200 mg/ml, preferably 150 mg/ml.

3. The pharmaceutical formulation of claim 1 or 2, wherein the poloxamer is present in a concentration of 0.02% - 0.06%, preferably 0.04 %.

4. The pharmaceutical formulation of claims 1 to 3, wherein the buffer is a sodium acetate buffer or a Histidine buffer, preferably a Histidine/Histidine-HCl buffer.

5. The pharmaceutical formulation of claims 1 to 4, wherein the buffer has a concentration of 10 to 30 mM, preferably 20 mM.

6. The pharmaceutical formulation of claims 1 to 5, wherein the pH of the formulation is 5 - 6, preferably 5.5.

7. The pharmaceutical formulation of claims 1 to 6, wherein the stabilizer is selected from sugars and amino acids.

8. The pharmaceutical formulation of claim 7, wherein the stabilizer is selected from trehalose and arginine.

9. The pharmaceutical formulation of claim 7 or 8, wherein the stabilizer has a concentration of 100 mM to 300 mM.

10. The pharmaceutical formulation of claim 8 or 9, wherein the stabilizer is trehalose and has a concentration of 150 mM to 250 mM, preferably 200 mM.

11. The pharmaceutical formulation of claim 8 or 9, wherein the stabilizer is arginine and has a concentration of 100 mM to 150 mM, preferably 135 mM.

12. The pharmaceutical formulation of claims 1 to 11, wherein the VH domain of the Abeta antibody comprises the amino acid sequence of Seq. Id. No. 2 and the VL domain of the Abeta antibody comprises the amino acid sequence of Seq. Id. No. 3.

13. The pharmaceutical formulation of claims 1 to12, wherein the heavy chain of the Abeta antibody comprises the amino acid sequence of Seq. Id. No. 10.

14. The pharmaceutical formulation of claims 1 to 13, wherein light chain of the Abeta antibody comprises the amino acid sequence of Seq. Id. No. 11.

15. The pharmaceutical formulation of claims 1 to 14, wherein the monoclonal Abeta antibody is a mixture of mono-glycosylated Abeta antibodies and double-glycosylated Abeta antibodies, wherein the mono-glycosylated antibody comprises a glycosylated asparagine (Asn) at position 52 of Seq. Id. No. 2 in the VH domain of one antibody binding site and wherein the double-glycosylated antibody comprises a glycosylated asparagine (Asn) at position 52 of Seq. Id. No. 2 in the VH domain of both antibody binding sites and whereby said mixture comprises less than 5% of an antibody being non-glycosylated at position 52 of Seq. Id. No. 2 in the VH domain.

**Patentansprüche**

1. Stabile flüssige pharmazeutische Antikörperformulierung, umfassend:

   - 50 mg/ml - 200 mg/ml eines Abeta-Antikörpers,
   - 0,01 % - 0,1% eines Poloxamers, bevorzugt Poloxamer 188,
   - 5 mM - 50 mM eines Puffers,
   - 100 mM - 300 mM eines Stabilisators,

   mit einem pH-Wert von 4,5 - 7,0,
   wobei der Abeta-Antikörper ein monoclonaler Antikörper ist, der eine schwere Kette und eine leichte Kette umfasst, wobei die schwere Kette des Abeta-Antikörpers eine VH-Domäne umfasst, umfassend:

   - eine CDR1, die die Aminosäuresequenz von SEQ ID NO:4 umfasst,
   - eine CDR2, die die Aminosäuresequenz von SEQ ID NO:5 umfasst,
   - eine CDR3-Sequenz, die die Aminosäuresequenz von SEQ ID NO:6 umfasst, und

   wobei die leichte Kette des Abeta-Antikörpers eine VL-Domäne umfasst, umfassend:

   - eine CDR1, die die Aminosäuresequenz von SEQ ID NO:7 umfasst,
   - eine CDR2, die die Aminosäuresequenz von SEQ ID NO:8 umfasst,
   - eine CDR3-Sequenz, die die Aminosäuresequenz von SEQ ID NO:9 umfasst.

2. Pharmazeutische Formulierung nach Anspruch 1, wobei die Abeta-Antikörperkonzentration 100 mg/ml - 200 mg/ml, bevorzugt 150 mg/ml, ist.

3. Pharmazeutische Formulierung nach Anspruch 1 oder 2, wobei das Poloxamer in einer Konzentration von 0,02% - 0,06%, bevorzugt 0,04%, vorliegt.

4. Pharmazeutische Formulierung nach den Ansprüchen 1 bis 3, wobei der Puffer ein Natriumacetat-Puffer oder ein Histidin-Puffer, bevorzugt ein Histidin/Histidin-HCl-Puffer, ist.

5. Pharmazeutische Formulierung nach den Ansprüchen 1 bis 4, wobei der Puffer eine Konzentration von 10 bis 30 mM, bevorzugt 20 mM, hat.

6. Pharmazeutische Formulierung nach den Ansprüchen 1 bis 5, wobei der pH-Wert der Formulierung 5 - 6, bevorzugt 5,5, ist.

7. Pharmazeutische Formulierung nach den Ansprüchen 1 bis 6, wobei der Stabilisator ausgewählt ist aus Zuckerarten und Aminosäuren.

8. Pharmazeutische Formulierung nach Anspruch 7, wobei der Stabilisator ausgewählt ist aus Trehalose und Arginin.

9. Pharmazeutische Formulierung nach Anspruch 7 oder 8, wobei der Stabilisator eine Konzentration von 100 mM bis 300 mM hat.

10. Pharmazeutische Formulierung nach Anspruch 8 oder 9, wobei der Stabilisator Trehalose ist und eine Konzentration von 150 mM bis 250 mM, bevorzugt 200 mM, hat.

11. Pharmazeutische Formulierung nach Anspruch 8 oder 9, wobei der Stabilisator Arginin ist und eine Konzentration von 100 mM bis 150 mM, bevorzugt 135 mM, hat.

12. Pharmazeutische Formulierung nach den Ansprüchen 1 bis 11, wobei die VH-Domäne des Abeta-Antikörpers die Aminosäuresequenz der SEQ ID NO:2 umfasst und die VL-Domäne des Abeta-Antikörpers die Aminosäuresequenz der SEQ ID NO:3 umfasst.

13. Pharmazeutische Formulierung nach den Ansprüchen 1 bis 12, wobei die schwere Kette des Abeta-Antikörpers die Aminosäuresequenz der SEQ ID NO:10 umfasst.

14. Pharmazeutische Formulierung nach den Ansprüchen 1 bis 13, wobei die leichte Kette des Abeta-Antikörpers die Aminosäuresequenz der SEQ ID NO:11 umfasst.

15. Pharmazeutische Formulierung nach den Ansprüchen 1 bis 14, wobei der monoclonale Abeta-Antikörper ein Gemisch aus einfach glycosylierten Abeta-Antikörpern und zweifach glycosylierten Abeta-Antikörpern ist, wobei der einfach glycosylierte Antikörper ein glycosyliertes Asparagin (Asn) an Position 52 der SEQ ID NO:2 in der VH-Domäne der einen Antikörperbindungsstelle umfasst und wobei der zweifach glycosylierte Antikörper ein glycosyliertes Asparagin (Asn) an Position 52 der SEQ ID NO:2 in der VH-Domäne beider Antikörperbindungsstellen umfasst und wobei das Gemisch weniger als 5% eines Antikörpers umfasst, der nicht an Position 52 der SEQ ID NO:2 in der VH-Domäne glycosyliert ist.

## Revendications

1. Formulation pharmaceutique liquide et stable d'anticorps comprenant :

   - 50 mg/ml - 200 mg/ml d'un anticorps abêta,
   - 0,01 % - 0,1% d'un poloxamère, de préférence d'un poloxamère 188,
   - 5 mM - 50 mM d'une solution tampon,
   - 100 mM - 300 mM d'un stabilisant,

   à un pH de 4,5 - 7,0,
   dans laquelle l'anticorps abêta est un anticorps monoclonal comprenant une chaîne lourde et une chaîne légère,
   dans laquelle la chaîne lourde de l'anticorps abêta comprend un domaine VH qui comprend :

   - une CDR1 comprenant la séquence d'acides aminés de la SEQ. ID. N° 4,
   - une CDR2 comprenant la séquence d'acides aminés de la SEQ. ID. N° 5,
   - une CDR3 comprenant la séquence d'acides aminés de la SEQ. ID. N° 6 et

   la chaîne légère de l'anticorps abêta comprend un domaine VL qui comprend :

   - une CDR1 comprenant la séquence d'acides aminés de la SEQ. ID. N° 7,
   - une CDR2 comprenant la séquence d'acides aminés de la SEQ. ID. N° 8,
   - une séquence CDR3 comprenant la séquence d'acides aminés de la SEQ. ID. N° 9.

2. Formulation pharmaceutique selon la revendication 1, dans laquelle la concentration d'anticorps abêta est de 100 mg/ml à 200 mg/ml, de préférence, de 150 mg/ml.

3. Formulation pharmaceutique selon la revendication 1 ou la revendication 2, dans laquelle le poloxamère est présent dans une concentration de 0,02 % à 0,06 %, de préférence, de 0,04 %.

4. Formulation pharmaceutique selon les revendications 1 à 3, dans laquelle la solution tampon est une solution tampon d'acétate de sodium ou une solution tampon d'histidine, de préférence, une solution tampon d'histidine/histidine-HCl.

5. Formulation pharmaceutique selon les revendications 1 à 4, dans laquelle la solution tampon a une concentration de 10 à 30 mM, de préférence, de 20 mM.

6. Formulation pharmaceutique selon les revendications 1 à 5, dans laquelle le pH de la formulation est de 5 à 6, de préférence, de 5,5.

7. Formulation pharmaceutique selon les revendications 1 à 6, dans laquelle le stabilisant est choisi parmi des sucres et acides aminés.

**8.** Formulation pharmaceutique selon la revendication 7, dans laquelle le stabilisant est choisi parmi le tréhalose et l'arginine.

**9.** Formulation pharmaceutique selon la revendication 7 ou la revendication 8, dans laquelle le stabilisant a une concentration de 100 mM à 300 mM.

**10.** Formulation pharmaceutique selon la revendication 8 ou la revendication 9, dans laquelle le stabilisant est du tréhalose et a une concentration de 150 mM à 250 mM, de préférence, de 200 mM.

**11.** Formulation pharmaceutique selon la revendication 8 ou la revendication 9, dans laquelle le stabilisant est de l'arginine et a une concentration de 100 mM à 150 mM, de préférence, de 135 mM.

**12.** Formulation pharmaceutique selon les revendications 1 à 11, dans laquelle le domaine VH de l'anticorps abêta comprend la séquence d'acides aminés de la SEQ. ID. N° 2 et le domaine VL de l'anticorps abêta comprend la séquence d'acides aminés de la SEQ. ID. N° 3.

**13.** Formulation pharmaceutique selon les revendications 1 à 12, dans laquelle la chaîne lourde de l'anticorps abêta comprend la séquence d'acides aminés de la SEQ. ID. N° 10.

**14.** Formulation pharmaceutique selon les revendications 1 à 13, dans laquelle la chaîne légère de l'anticorps abêta comprend la séquence d'acides aminés de la SEQ. ID. N° 11.

**15.** Formulation pharmaceutique selon les revendications 1 à 14, dans laquelle l'anticorps abêta monoclonal est un mélange d'anticorps abêta mono-glycosylés et d'anticorps abêta di-glycosylés, dans laquelle l'anticorps mono-glycosylé comprend une asparagine glycosylée (Asn) à la position 52 de la SEQ. ID. N° 2 dans le domaine VH d'un site de liaison à l'anticorps et dans laquelle l'anticorps diglycosylé comprend une asparagine glycosylée (Asn) à la position 52 de la SEQ. ID. N° 2 dans le domaine VH des deux sites de liaison à l'anticorps, ledit mélange comprenant moins de 5 % d'un anticorps étant non-glycosylé à la position 52 de la SEQ. ID. N° 2 dans le domaine VH.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008071394 A
- US 5202238 A
- US 5204244 A
- US 4816567 A
- US 5648237 A
- US 5789199 A
- US 5840523 A
- US 5959177 A
- US 6040498 A
- US 6420548 B
- US 7125978 B
- US 6417429 B
- WO 2007068429 A
- EP 12158602 A

**Non-patent literature cited in the description**

- **MANNING, M. C. ; K. PATEL et al.** Stability of protein pharmaceuticals. *Pharm Res,* 1989, vol. 6 (11), 903-18
- **ZHENG, J. Y. ; L. J. JANIS.** Influence of pH, buffer species, and storage temperature on physicochemical stability of a humanized monoclonal antibody LA298. *Int)_Pharm.,* 2005
- **SHIRE, S. J. ; Z. SHAHROKH et al.** Challenges in the development of high protein concentration formulations. *J Pharm Sci,* 2004, vol. 93 (6), 1390-402
- **ROSKOS, L. K. ; C. G. DAVIS et al.** The clinical pharmacology of therapeutic monoclonal antibodies. *Drug Development Research,* 2004, vol. 61 (3), 108-120
- **SHIRE, S.1. ; Z. SHAHROKH et al.** Challenges in the development of high protein concentration formulations. *J Pharm Sci,* 2004, vol. 93 (6), 1390-402
- **HOUSTON, J.S.** *Methods in Enzymol.,* 1991, vol. 203, 46-96
- **MORRISON, S.L. et al.** *Proc. Natl. Acad. Sci. USA,* 1984, vol. 81, 6851-6855
- **RIECHMANN, L. et al.** *Nature,* 1988, vol. 332, 323-327
- **NEUBERGER, M.S. et al.** *Nature,* 1985, vol. 314, 268-270
- **VAN DIJK, M.A. ; VAN DE WINKEL, J.G.** *Curr. Opin. Chem. Biol.,* 2001, vol. 5, 368-374
- **JAKOBOVITS, A. et al.** *Proc. Natl. Acad. Sci. USA,* 1993, vol. 90, 2551-2555
- **JAKOBOVITS, A. et al.** *Nature,* 1993, vol. 362, 255-258
- **BRUGGEMANN, M. et al.** *Year Immunol.,* 1993, vol. 7, 33-40
- **HOOGENBOOM, H.R. ; WINTER, G.** *J. Mol. Biol.,* 1992, vol. 227, 381-388
- **MARKS, J.D. et al.** *J. Mol. Biol.,* 1991, vol. 222, 581-597
- **COLE et al.** Monoclonal Antibodies and Cancer Therapy. Alan R. Liss, 1985, 77
- **BOERNER, P. et al.** *J. Immunol.,* 1991, vol. 147, 86-95
- **KABAT et al.** Sequences of Proteins of Immunological Interest. Public Health Service, National Institutes of Health, 1991
- **CLELAND, J. L. ; M. F. POWELL et al.** The development of stable protein formulations: a close look at protein aggregation, deamidation, and oxidation. *Crit Rev Ther Drug Carrier Syst,* 1993, vol. 10 (4), 307-77
- **WANG, W.** Instability, stabilization, and formulation of liquid protein pharmaceuticals. *Int J Pharm,* 1999, vol. 185 (2), 129-88
- **WANG, W.** Lyophilization and development of solid protein pharmaceuticals. *Int J Pharm,* 2000, vol. 203 (1-2), 1-60
- **CHI, E. Y. ; S. KRISHNAN.** Physical stability of proteins in aqueous solution: mechanism and driving forces in nonnative protein aggregation. *Pharm Res,* 2003, vol. 20 (9), 1325-36
- **CHARLTON.** Methods in Molecular Biology. Humana Press, 2003, vol. 248, 245-254
- **GERNGROSS.** *Nat. Biotech.,* 2004, vol. 22, 1409-1414
- **LI et al.** *Nat. Biotech.,* 2006, vol. 24, 210-215
- **GRAHAM et al.** *J. Gen Virol.,* 1977, vol. 36, 59
- **MATHER.** *Biol. Reprod.,* 1980, vol. 23, 243-251
- **MATHER et al.** Annals N.Y. Acad. Sci. 1982, vol. 383, 44-68
- **URLAUB et al.** *Proc. Natl. Acad. Sci. USA,* 1980, vol. 77, 4216
- **YAZAKI ; WU.** Methods in Molecular Biology. Humana Press, 2003, vol. 248, 255-268